(19) Europäisches Patentamt
European Patent Office
Office européen des brevets

(11) Publication number: **0 200 211**
**A1**

# (12) EUROPEAN PATENT APPLICATION

(21) Application number: 86105920.2

(22) Date of filing: 29.04.86

(51) Int. Cl.[4]: C 07 C 149/24
C 07 D 285/00, C 07 F 13/00
A 61 K 49/02

(30) Priority: 01.05.85 US 729300

(43) Date of publication of application:
05.11.86 Bulletin 86/45

(84) Designated Contracting States:
AT BE CH DE FR GB IT LI LU NL SE

(71) Applicant: THE RESEARCH FOUNDATION OF STATE UNIVERSITY OF NEW YORK
State University Plaza Broadway
Albany New York 12246(US)

(72) Inventor: Kung, Hank Fan-luan
192 Surrey Run
Williamsville New York 14221(US)

(72) Inventor: Efange, Simon Mbua Ngale
7919 Southwest 104th Street
Miami Florida 33156(US)

(72) Inventor: Blau, Monte
P.O. Box 605
S. Wellfleet Massachusetts 02663(US)

(72) Inventor: Yu, Chi-Chou
470 Washington Street
Canton Massachusetts 02021(US)

(74) Representative: Weber, Dieter, Dr. et al,
Dr. Dieter Weber und Klaus Seiffert Patentanwälte
Gustav-Freytag-Strasse 25 Postfach 6145
D-6200 Wiesbaden 1(DE)

(54) **Diagnostic radiopharmaceutical compounds.**

(57) A radiopharmaceutical chemical compound is claimed comprising radioactive Technetium-99m having the structure:

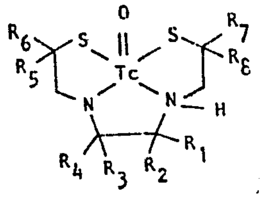

wherein each $R_1$, $R_2$, $R_3$, $R_4$, $R_5$, $R_6$, $R_7$ and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; provided at least one of $R_1$, $R_2$, $R_3$, $R_4$, $R_5$, $R_6$, $R_7$, and $R_8$, contains at least one nitrogen atom. The non-isotope containing ligand precursor compounds and the diene intermediate to the ligand are also claimed.

Compounds of this structure comprising Technetium-99m have a lipophilicity sufficiently high at a pH of 7.6 to permit passage of the compounds from the blood of a mammal into a target organ or tissue and sufficiently low at a pH of 6.6 to prevent rapid return of the compounds from the target organ or tissue to the blood.

A method and kit for selectively depositing a radiopharmaceutical compound in at least one target tissue or organ of a mammal, which tissue or organ has a significantly different intracellular pH than the blood of the mammal, by introducing one or more compounds of the invention into the bloodstream of the mammal.



Croydon Printing Company Ltd.

# DIAGNOSTIC RADIOPHARMACEUTICAL COMPOUNDS

This invention was made with Government support under Grant Number 1 R01 NS18509 awarded by the Department of Health and Human Services. The Government has certain rights in this invention.

## BACKGROUND OF THE INVENTION

### A) Field of the Invention

The present invention is directed to new diene compounds, new ligands, new technetium-99m radiopharmaceutical compounds which are derived therefrom, and to a method and kit for selectively depositing, for diagnostic purposes, technetium-99m containing radiopharmaceutical compounds in target tissues or organs of a mammal. Mammal as used herein includes the human being. The invention more particularly relates to new diene compounds which are intermediate in the production of new ligands, which have utility as chelating agents, and technetium-99m containing radiopharmaceutical compounds which are capable of selective accumulation in tissues or organs having lowered intracellular pH as a result of normal metabolism or diseased state.

### B) Description of the Prior Art

Radiopharmaceutical compounds have been in use for diagnostic purposes for a long time. Those well versed in the

art relating to radiopharmaceuticals and nuclear medicine are well aware of the requirements which must be satisfied by a diagnostically useful radiopharmaceutical compound. Briefly, these requirements include the following. The radio-pharamaceutical compound must be able to penetrate into a target tissue or organ and attain a sufficiently high concentration therein so that its presence is detectable by state of the art radiation monitoring means. The accumulation of the radiopharmaceutical compound in the target tissue or organ must be sufficiently selective relative to other tissues and organs of the body so that a diagnostic distinction for its presence in the target tissue or organ relative to the other tissues or organs can be made. Furthermore, the radiopharmaceutical compound must emit radiation capable of penetrating through several other tissues or organs of the body. Experience has shown that only radiopharmaceutical compounds emitting gamma, X-ray or positron radiation satisfy this requirement. Finally, and preferably, a diagnostic radiopharmaceutical compound should be easily prepared from inexpensive and available radionuclides.

U.S. Patents 4,363,793 and 4,474,746 describe in detail the various prior art means to attain tissue and/or organ penetration and uptake by a radiopharmaceutical compound by exploiting various differences in metabolic or physiological states of the several tissues of the body, including methods which take advantage of lipid solubility and regional pH differences with an organ which allows selective radioisotope deposition utilizing

regional pH shift. For further information on these phenomena, see an article by Michael D. Loberg et al, entitled "Membrane Transport of Tc-99m-Labeled Radiopharmaceuticals Initial Brain Uptake by Passive Transport", J. Nucl. Med. Vol. 20: No. 11, pp. 1181-1188 and an article by W. J. Waddell and R. G. Bates titled "Intracellular pH," Physiological Review 49: pp. 286-329, 1969.

The method disclosed in U.S. Patents 4,363,793 and 4,474,746 exploiting required pH shift to target radiopharmaceutical compounds has proven to be a valuable diagnostic tool but the compounds identified by the patents for attaining such method have not been as effective as desired. Thus, a need exists for new radiopharmaceutical compounds which exhibit a full complement of desirable characteristics, particularly for single photon emission tomography, such as high initial brain uptake, fixed regional distribution, long brain retention, and a high brain/blood ratio.

## SUMMARY OF THE INVENTION

In accordance with the present invention, new diene compounds have been provided which can be readily reduced to form new ligand compounds which have broad utility as metal chelating agents and which when combined with technetium-99m isotope form new technetium-99m containing diagnostic radiopharmaceutical compounds which are capable of entering a target tissue or a target organ by passive diffusion through cell walls and which are effectively accumulated and retained within the target tissue or organ due to a regional pH shift. Such compounds can be

readily produced by a method of the invention and have displayed a full complement of metal chelating and diagnostically desirable characteristics.

More specifically, the new diene compounds of the invention are of the formula:

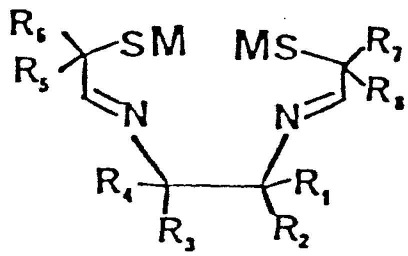

wherein each of $R_1$, $R_2$, $R_3$, $R_4$, $R_5$, $R_6$, $R_7$, and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; each M is selected from hydrogen and substituted or unsubstituted alkyl, alkene, acyl alkyl, acyl aryl, alkoxy alkyl, alkoxy aryl, acyl alkoxy, acyl aryloxy, silyl trialkyl, alkyl aryl and the like; or M represents a covalent bond between the two sulfur atoms of the structure; provided at least one of $R_1$, $R_2$, $R_3$, $R_4$, $R_5$ $R_6$, $R_7$, and $R_8$ contains at least one nitrogen atom.

The ligand compounds of the invention are of the formula:

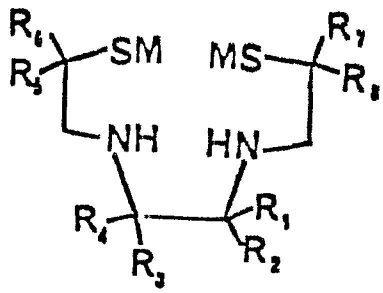

wherein each of $R_1$, $R_2$, $R_3$, $R_4$, $R_5$, $R_6$, $R_7$, and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; each M is selected from hydrogen and substituted or unsubstituted alkyl, alkene, acyl alkyl, acyl aryl, alkoxy alkyl, alkoxy aryl, acyl alkoxy, acyl aryloxy, silyl trialkyl, alkylaryl and the like, or M represents a covalent bond between the two sulfur atoms of the structure; provided at least one of $R_1$, $R_2$, $R_3$, $R_4$, $R_5$, $R_6$, $R_7$, and $R_8$ contains at least one nitrogen atom.

The radiopharmaceutical compounds of the invention are of the formula:

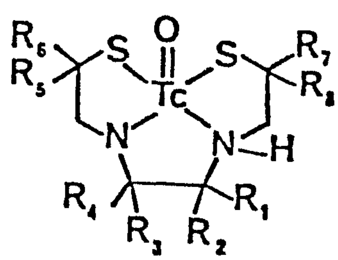

wherein each of $R_1$, $R_2$, $R_3$, $R_4$, $R_5$, $R_6$, $R_7$, and $R_8$, is independently selected from at least one of hydrogen and

substituted or unsubstituted alkyl, alkenyl, alkynyl, cycloalkyl, heterocyclic alkyl, aryl, carboxylic acid ester, and carboxylic acid amide; provided at least one of $R_1$, $R_2$, $R_3$, $R_4$, $R_5$, $R_6$, $R_7$, and $R_8$ contains at least one nitrogen atom.

The technetium-99m radioactive element of the structure is an element which emits gamma ray (gamma-ray) at a half life of about 6 hours and gamma energy of about 140 kiloelectron volts (KeV) which is detectable in a target organ or tissue having a significantly different and usually lower intracellular pH than the blood of the mammal. The lipophilicity is sufficiently low at a pH of 6.6 or lower and usually at 7.0 or lower to prevent rapid return of the compound from the target organ or tissue to the blood. The compounds of the pharmaceutical method further will have no overall net positive charge at pH 12 and no net overall negative charge at pH 6.0. The compounds desirably have an octanol-aqueous medium partition coefficient which is significantly dependent on the pH of the aqueous medium at least in the pH range of 6.60 to 7.60.

In a method of the invention, the compounds are introduced into the blood circulation of a mammal, which as previously mentioned includes human beings, wherein they are in a relatively lipid soluble state. The compounds readily penetrate several tissues and organs of the body by passive diffusion. In target tissues or organs of the body wherein the intracellular pH is significantly lower than the pH of the bloodstream, the compounds assume a more hydrophilic, less lipid soluble state whereby the

rate of egress of the compounds from the cells by diffusion is significantly diminished. As a result, the compounds are at least temporarily trapped within the target tissue or organ and may be detected therein by the radiation monitoring means.

BRIEF DESCRIPTION OF THE DRAWINGS

Figure 1 is an autoradiogram of rat brain distribution of radioactivity two (2) minutes after intravenous injection with a radioactive compound of the prior art.

Figure 2 is an autoradiogram of rat brain distribution of radioactivity fifteen (15) minutes after intravenous injection with a radioactive compound of the prior art.

Figure 3 is an autoradiogram of rat brain distribution of radioactivity two (2) minutes after intravenous injection with a radioactive compound of the invention.

Figure 4 is an autoradiogram of rat brain distribution of radioactivity fifteen (15) minutes after intravenous injection with a radioactive compound of the invention.

DETAILED DESCRIPTION OF THE INVENTION

In accordance with the present invention, radiopharmaceutical compounds of the formula:

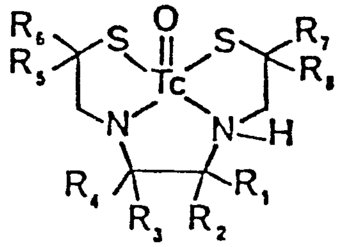

wherein each of $R_1$, $R_2$, $R_3$, $R_4$, $R_5$, $R_6$, $R_7$, and $R_8$ as previously described can be prepared by a novel radiosynthetic process, wherein an appropriate $\alpha,\alpha'$-dithio substituted or unsubstituted dialkyl aldehyde is reacted with an appropriate substituted or unsubstituted alkyl vicinal diamine to form the 1,2-dithio-5,8-diazacyclodeca-4,8-diene-3,3',6,6',7,7',10,10'- substituted or unsubstituted alkyl amide and/or amine in accord with the following:

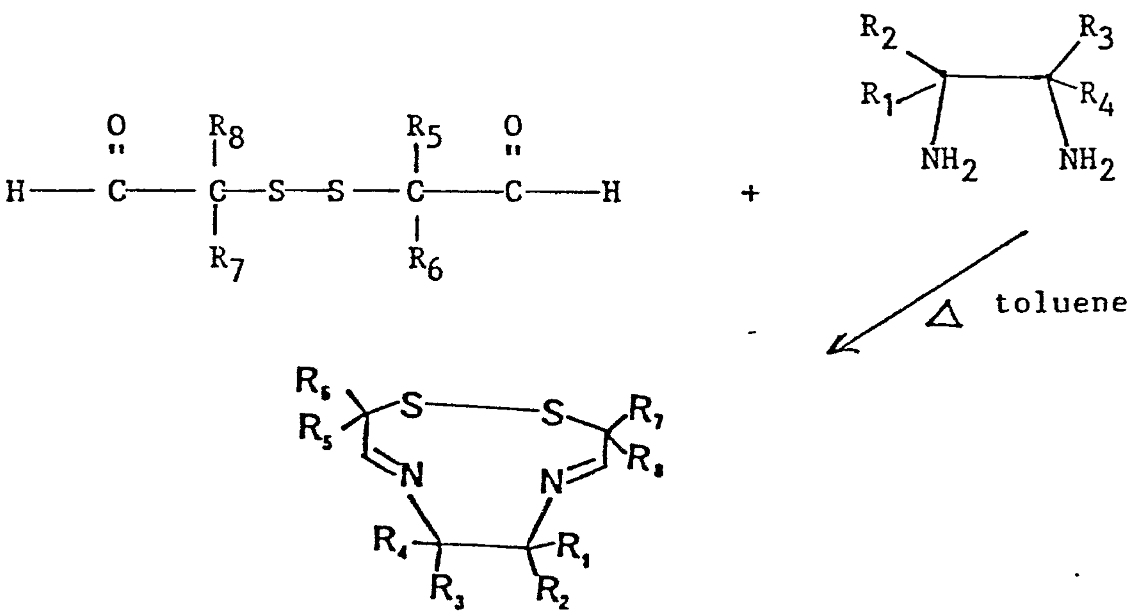


Wherein $R_1$ - $R_8$ are as before described. The 1,2-dithio-5,8-diazacyclodeca-4,8-diene-3,3',6,6',7,7',10,10'-substituted or unsubstituted alkyl amide and/or amine is readily reduced to form the 1,2-dithio-5,8-diazacyclodeca-3,6,7,10-substituted or unsubstituted amine:

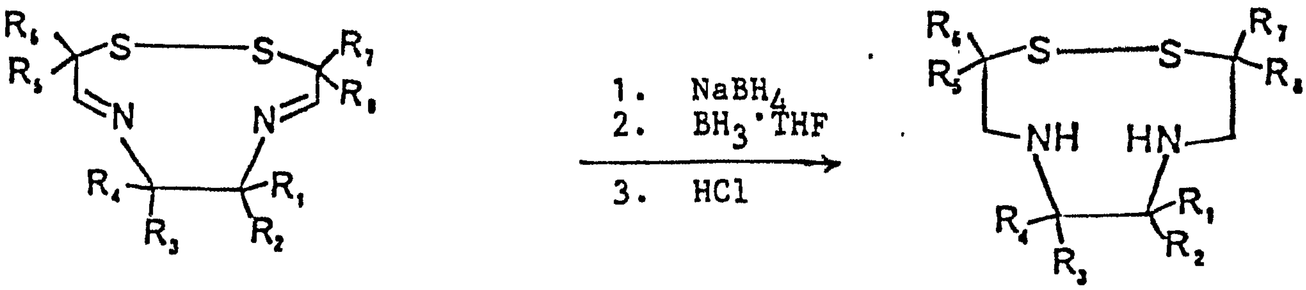


Labeling of the 1,2-dithio-5,8-diazocyclodeca-3,3',6,6',7,7',10,10'-substituted or unsubstituted alkyl amine is achieved by reducing technetium pertechnetate, preferably with either sodium borohydride or stannous pyrophosphate in ethanol or saline, in the presence of the compound. The disulfide bond of the 1,2-dithio-5,8-diazacyclodeca-substituted or unsubstituted dialkyl amine is reduced along with the pertechnetate to produce the desired technetium complex in accordance with the following:

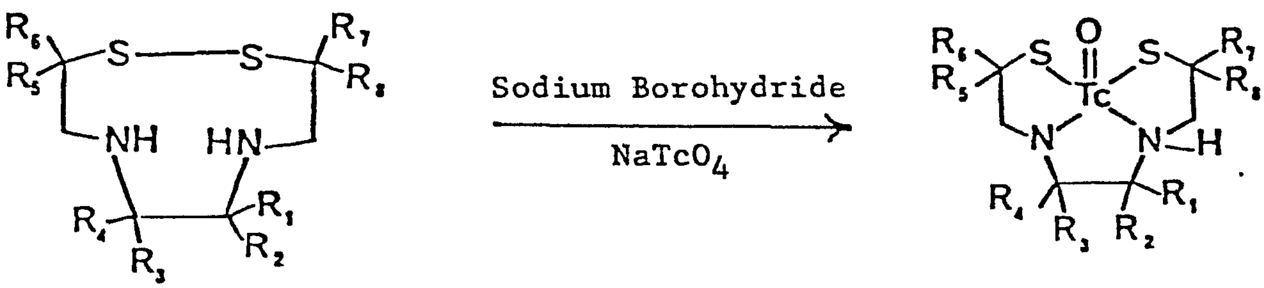


In certain instances it may be desirable to reduce the disulfide bond prior to treatment with an appropriate technetium-99m source.

Generally, the $\alpha$, $\alpha'$-dithio substituted or unsubstituted dialkyl aldehydes utilized as a starting material for the aforesaid method of preparation can be prepared by various means

known in the prior art. One such method for manufacturing the symmetrical dialdehyde comprises, reaction of an appropriate substituted or unsubstituted aldehyde with $S_2Cl_2$ (sulfuryl chloride) in accord with the following reaction scheme:

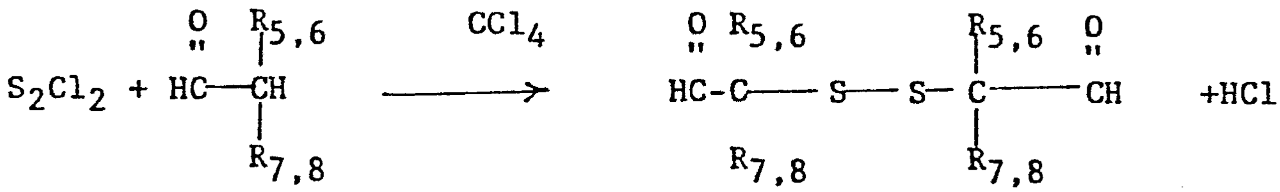


wherein $R_5$ - $R_8$ are as previously described. In instances wherein $R_5$ - $R_8$ contain aryl or other easily chlorinated reactive groups, it may be necessary to follow an alternative synthetic sequence to avoid chlorination of the substituent. Such method, as described, can also be utilized to form the unsymmetrical aldehyde, however, the multiple variable products which result require sophisticated separation techniques.

We have found that alternately, the asymmetric substituted compounds of the invention can be easily produced by one of skill in the art by sequentially linking each of the vicinal nitrogen of an appropriate substituted or unsubstituted diamine with an appropriately S-substituted thioglycolic acid in accord with the following, where X is a leaving group such as halogen and substituted or unsubstituted alkoxy, aryloxy, n-oxyimide and the like, $R_1$-$R_8$ are as previously described and M is substituted or unsubstituted alkyl, alkene, acyl alkyl, acyl aryl, alkoxy alkyl, alkoxy aryl, acyl alkoxy, acyl aryloxy, silyl trialkyl, alkyl aryl and the like:

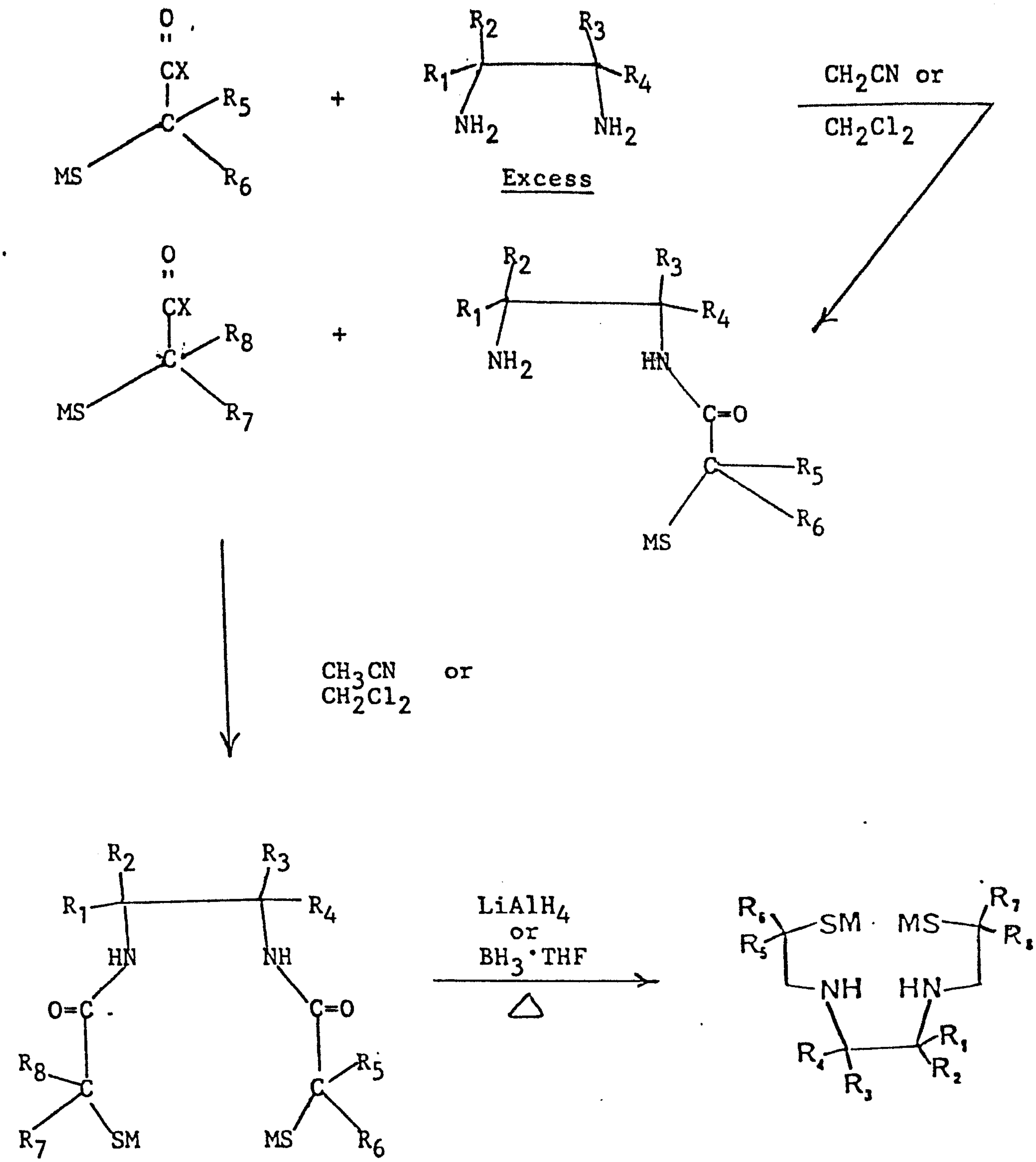
Excess
CH2CN or
CH2Cl2
CH3CN or
CH2Cl2
LiAlH4
or
BH3·THF

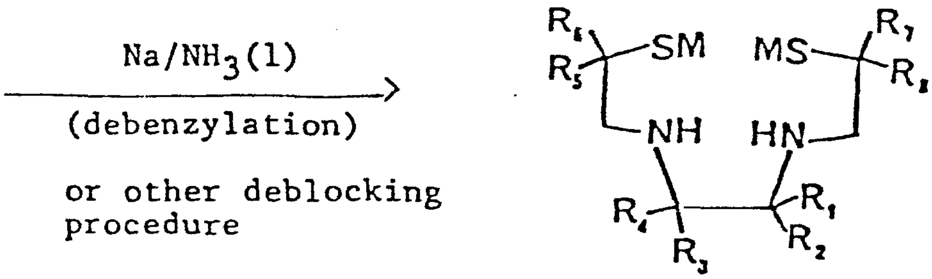


The alkyl, alkene, acyl alkyl, acyl aryl, alkoxy alkyl, alkoxy aryl, acyl alkoxy, acyl aryloxy, silyl trialkyl, alkyl aryl substituents of M act to block the sulfur atom from reactivity and are replaced with hydrogen to provide the active ligand chelating agent which can then form the radiopharmaceutical compounds of the invention. The resulting ligand can be easily labelled with an appropriate technetium-99m isotope by treatment with reactant technetium pertechnetate. "Technetium" as used herein is intended to mean the technetium-99m isotope.

Appropriately S-substituted thioglycolic acids such as used in the aforesaid reaction scheme are commercially available or can be easily prepared by prior art means such as by sequential alkylation using an appropriate substituted or unsubstituted alkyl halide. For further information on the manufacture of various S-substituted thioglycolic acids see: J. Amer. Chem. Soc. 15, 201 (1963); Chem. Pharm. Bulletin 26, 1576 (978); Organic Synthesis Collective, Vol. IV, p. 291; J. Med. Chem. 6, 136 (1963).

Generally, the substituted or unsubstituted vicinal diamines used as a reactant to form the organic compounds of the invention are commercially available. Various means are known in the prior art for the manufacture of these compounds wherein at least one of $R_1$ - $R_4$ is other than hydrogen, one such method comprising derivation through an appropriate carboxylate substituted ethylene diamine of the formula:

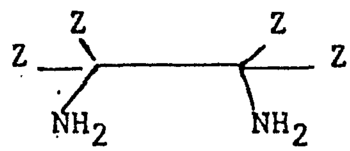

wherein Z is carboxylate or hydrogen and at least one Z is carboxylate. Typically, to block undesired reactivity at the amine sites, the diamine is first protected with a reagent such as di-tert-butyldicarbonate acid to form a 2,3 bis(t-butoxycarbamoyl) propanoate. The carboxylate radical of the blocked diamine can be easily hydrolized with a strong base, such as potassium hydroxide, to produce the protected propanoic acid which can thereafter be reacted with an appropriate hydroxy $R_1$ - $R_4$ group to position the appropriate substituent.

For further information on the manufacture of various vicinal diamines see Harold Kohn et al, Amer. Chem. Soc. Vol. 105, No. 12, 1983, "New Stereoselective Method for the Preparation of Vicinal Diamines from Olefins and Cyanamide", pp. 4106-4108; and, Natsugari et al, J. Amer. Chem. Soc. Vol. 106, No. 25, 1984, "Stereocontrolled Synthesis of Unsaturated Vicinal Diamines from Diels-Alder Adducts of Sulfur Dioxide Bis (Imides)", pp. 7867-7872.

Within the description of $R_1$ - $R_8$, by the term alkyl, alkenyl and alkynyl is meant alkyl, alkenyl and alkynyl hydrocarbon substituents having from 1 to about 20 carbon atoms and preferably from 1 to about 10 carbon atoms. Such substituents can be straight chained, branched,cyclic and include isomers thereof. Thus, the term alkyl includes methyl, ethyl, n-propyl, i-propyl, n-butyl, s-butyl, t-butyl, i-butyl, pentyl, hexyl, heptyl and the like up to about 20 carbon atoms. Similarly the terms alkenyl and alkynyl include unsaturated hydrocarbons having double or triple bonds therein such as ethene, propene, butene, pentene and acetylene, methylacetylene, ethylacetylene and the like up to about 20 carbon atoms. By the term cyclic is meant the alicyclic saturated or unsaturated hydrocarbons of up to about 20 carbon atoms such as cyclopropyl, methylcyclopropyl, cyclobutyl, ethylcyclobutyl, cyclopentyl, cyclohexyl, cyclohexenyl, cyclohexadienyl, cyclooctynyl and the like. By the term heterocyclic alkyl is meant a cyclic hydrocarbon compound containing a noncarbon atom as part of the cyclic structure. Typically such noncarbon atoms are nitrogen, phosphorus, sulfur or oxygen, with the heterocyclic containing up to about 20 carbon atoms with the attachment to the basic structure through a carbon atom and never through a noncarbon atom. Typical examples of appropriate heterocyclic alkyl substituents include piperidinyl, pyrrolyl, pyrrolizinyl, pyridyl, imidazolyl, furfuryl, morpholinyl, piperazyl, thiazolyl, thiomorpholinyl, tetrahydroquinolinyl, oxazolyl, azepinyl,

indoxyl, indolizinyl and the like. By the term aryl, is meant those cyclic aromatic and heteroaromatic structures which include benzene, naphthalene, pyridine, pyrimidine, quinoline, thiopheneindole, phenanthrene, anthracene, etc., up to a total of about 20 carbon atoms. The preferred aryl substituents are phenyl and napthalalyl.

By the term carboxylic acid ester is meant any ester which can be derived through a carboxylic acid and generally includes compounds of the structure

$$R_n-\overset{O}{\overset{\|}{C}}-O-R$$

wherein each R is substituted or unsubstituted hydrocarbon up to a total of about 20 carbon atoms and n is 0 or 1. Generally such esters can comprise straight hydrocarbon chains or branched and include the isomers thereof. Attachment can, of course, occur though the R group or directly to the carbonyl by loss of R group substituents. Typical examples include:

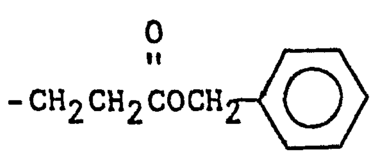 ,

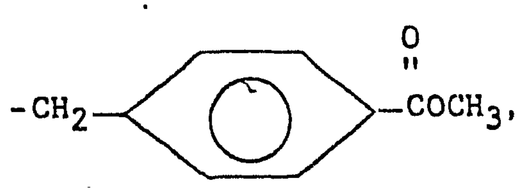

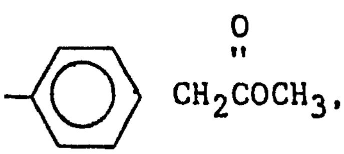

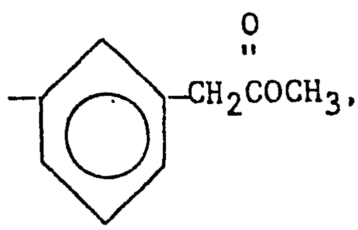

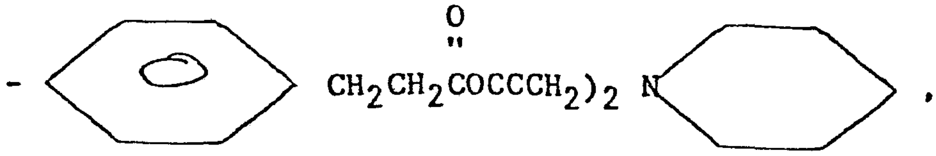

$-(CH_2)_2\overset{O}{\overset{\|}{C}}OCH_2CH_3$,

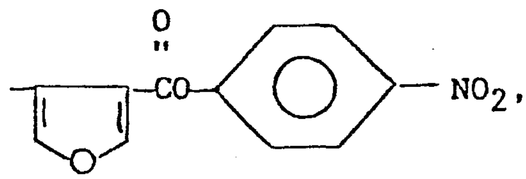

$-\overset{O}{\overset{\|}{C}}OCH(CH_3)_2$,

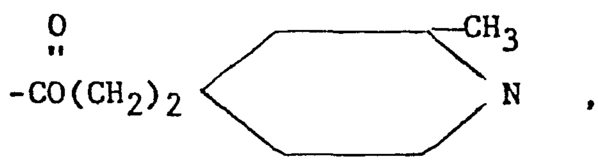

$-\overset{O}{\overset{\|}{C}}OCH_2C(CH_3)_3$, and the like.

By the term carboxylic acid amide, is meant a compound having the general formula $RCONH_2$, $RCONHR$ or $RCONR_2$, wherein R is a hydrocarbon up to about 20 carbon atoms. Typically such amides can comprise straight hydrocarbon chains or branched and include the isomers thereof. typical examples include

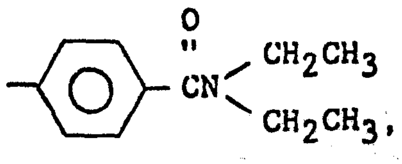

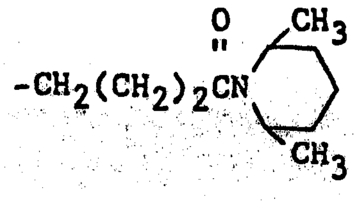

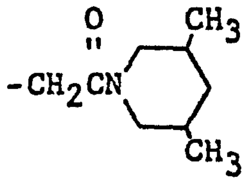

,

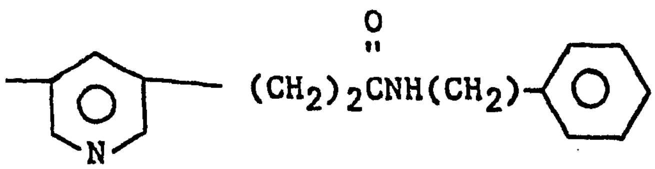

and the like.

Each of the aforesaid primary $R_1$ - $R_8$ substitutents can also be substituted with one or more substitu ents selected from alkoxy, haloaryl, aryl, aryl amino, cyano, alkyl amino, alkyl thio, alkyl ester, aryl ester, nitro aryl, alkenyl, alkynyl and alkyl, providing that the total number of carbon atoms does not exceed about 20 and preferably about 10.

Within the description of M, the terms alkyl and alkene are meant to include alkyl and alkoxy substituents having from 1 to about 20 carbon atoms and preferably from 1 to about 10 carbon atoms. Such substituents can be straight chained, branched or cyclic and include isomers thereto. Thus, the term alkyl includes methyl, ethyl, n-propyl, i-propyl, n-butyl, s-butyl, pentyl, hexyl, cy clohexyl and the like, up to about 20 carbon atoms; while, the term alkene includes unsaturated hydrocarbons having one or more double bonds therein and includes ether,

propene, butene, pentene and the like. The terms acyl alkyl, and acyl aryl are meant to include an alkyl or aryl substituent which is not directly bonded to the sulfur but is bonded through a carbonyl group. Examples of such groups include acetyl, benzoyl and the like. The terms alkoxy alkyl and alkoxy aryl are meant to include an alkyl or aryl substituent containing an ether linkage between two carbons, wherein the sulfur atom is bonded to a carbon atom, and includes substituents such as methoxy methyl, phenoxy methyl and the like. Acyl alkoxy and acyl aryloxy are meant to include an alkoxy or aryloxy substituent bonded to the sulfur through a carbonyl group and include substituents such as t-butoxycarbonyl, benzyloxycarbonyl and the like. Silyl trialkyl is meant to include the trisubstituted silyl groups such as trimethylsilyl, t-butyldimethylsilyl and the like. The term alkyl aryl is meant to include aryl substituents bonded to the sulfur atom through an alkyl group.

In accordance with the present invention, radioactively labeled chemical compounds which preferentially accumulate in certain target tissues or organs of the body are prepared and introduced into the blood stream of a mammal, including a human being.

The manner of introduction into the blood stream can be any convenient method well known to those of skill in the art. Typically, two basic methods are used, intravenous or intraarterial injection. Generally, in either method, the radioactive compound of the invention would be directly injected as an

isotonic solution preferably saline or buffered saline. Depending upon the target region gamma camera imaging would begin immediately or after an appropriate waiting period for optimum target to background ratio.

The normal manner in which it is contemplated to supply the compounds of the invention for regular nuclear medicine application is as a lyophilized kit. In such form the active ingredients will comprise the claimed ligand compound in isotonic solution, with or without an appropriate buffer and reducing agent, lyophilized. Lyophilization of a compound in isotonic solution is well known in the prior art. The buffer can be any appropriate buffering compound such as various of the sodium or potassium phosphates. The reducing agent can be any compound that will reduce the technetium isotope. Preferably, the reducing agent is stannous chloride or similar FDA approved agent. The lyophilized pharmaceutical composition is typically supplied to the nuclear medicine facility which then adds the technetium-99m isotope to form the pharmaceutical compounds of the invention. The preferred chemical form of the isotope is technetium-99m pertechnetate.

The target tissues or organs of the body, in accordance with the present invention, are tissues or organs which show a regional pH shift relative to surrounding tissues, organs or the bloodstream. As it was briefly discussed above, medical science has established that such regional pH shifts exist in the brain as well as in certain tumors and in ischemic heart tissue. Such pH shifts may also occur in various forms of abscesses. Since tumors may be entirely surrounded by healthy tissue of an organ, regional pH shifts may exist between healthy and malignant tissues of the same organ. The diagnostic significance of preferential accumulation of a radiopharmaceutical compound in a selected target organ or within the abnormal tissue of an organ is readily apparent to those skilled in the medical arts, and need not be further elaborated here.

Monitoring or observing the selective accumulation of radiopharmaceutical compounds in the target tissues or organs may be readily accomplished by currently available radioactive imaging or scanning techniques and instruments. The amount of radioactive labeled compound or radiopharmaceuticl absorbed within the selected target tissue or organ may be expressed in terms of percentage of a total dose of the radiopharmaceutical compound which is injected into the animal.

Radiopharmaceutical compounds which may be used in the novel diagnostic method of the present invention must satisfy the following requirements. They must possess lipid solubility characteristics significantly dependent on pH in at least the 6.6

to 7.6 pH range and desirably in the 7.0 to 7.4 pH range. Since the regional pH shift in all of the presently known target organs or tissues is toward a pH lower than the normal pH of the blood (approximately 7.4), the compounds of the present invention must show increasing lipid solubility and therefore increasing cell wall penetrating capability with increasing pH, at least in the 6.60 to 7.60 pH range. In addition, the compounds of the present invention must also emit gamma, X-ray or positron radiation which can penetrate through surrounding organs and tissues of the body for ultimate detection by suitable monitoring instruments. Most such desirable compounds emit gamma or positron radiation.

It is an important aspect of the present invention that the herein described radiopharmaceutical compounds penetrate through the cell walls by passive diffusion rather than being dependent on a specialized active or facilitated transport mechanism. The advantage of the ability to penetrate into the cells by diffusion is that the selective accumulation of a radiopharmaceutical compound of the present invention is principally dependent on the intracellular pH of the target, rather than on close structural similarity to a metabolite for which an active or facilitated transport mechanism exists. As is well known in the biochemical and related arts, the requirement for structural similarity between an analog and a metabolite are very exacting for the analog to be actively transported into the cells. Thus, compounds of the present invention may be of a wide variety of chemical structures; the principal limiting requirement being the presence

of the requisite lipid solubility-pH dependence characteristics. After having penetrated the target having a regional pH shift, the compound is effectively trapped in the cells of the target because it is less lipid soluble at the pH of the target than at the pH of the blood. Consequently, the rate of egress of the compound by passive diffusion from the target is significantly slower than its rate of entry, i.e., the rapid return of the compound to the blood is prevented, which results in accumulation of the compound in the target, e.g., the egress or return rate may be from 2 to more than 100 times slower than the entry rate and is thus relatively less rapid than the entry rate. To "prevent rapid return" as used throughout the specification and claims is relative and means that the relative rate of return to the blood from the organ or tissue is slower than the entry into the organ or tissue from the blood as previously discussed.

It has been found that conventional n-octanol-aqueous medium provide exellent indicia to determine the sufficient lipophilicity of a radiopharmaceutical compound at pH 7.6 to permit passage of the compound from the blood into the target organ or tissue and the insufficient lipophilicity to prevent rapid return of the compound from the target organ or tissue to the blood at pH 6.6. The n-octanol-aqueous medium partition coefficients of the compound are readily measured in accordance with standard practice in the prior art. Briefly, such measurements include the steps of dissolving a known amount of the compound in an aqueous buffer of a predetermined pH and

extracting the buffer solution with a known amount of n-octanol until an equilibrium of distribution of the compound between the aqueous and n-octanol phases occurs. The concentration of the compound in both phases is then measured by suitable analytical means, such as ultraviolet spectrophotometry; the partition coefficient being the ratio of the two measured concentrations.

For the purposes of evaluating a plurality of radioactive compounds for use in the novel method of the present invention, the n-octanol-aqueous medium partition coefficients of the compounds were measured in the following manner. The radioactive compound was mixed with 1.0 ml of n-octanol and 1.0 ml of buffer of the desired predetermined pH. The radioactivity of this mixture was counted, and the mixture was placed in a water bath shaker at 37°C for 2 hours. After centrifugation at 3,000 rpm for 5 minutes, the n-octanol layer was separated and its radioactivity counted. The partition coefficient of the compound was calculated by the following equation:

$$\text{Partition Coefficient} = \frac{\text{counts in n-octanol}}{\text{initial counts-counts in n-octanol}} \quad \text{(Equation 1)}$$

Partition coefficients obtained in this manner for a plurality of radiopharmaceutical compounds of the present invention, are shown in Table I.

It has been found that compounds which are suitable for use in the novel diagnostic method of the present invention have n-octanol-aqueous medium partition coefficients (hereinafter referred to simply as partition coefficients) which increase by

at least 20% as the pH increases from 6.60 to 7.60. Preferably the partition coefficient of the compound increases by at least 50% in the pH range from 6.60 to 7.60. The preferred partition coefficient at pH 7.6 for a particular compound is often between 0.5 and 500.

The isotope in general is usually chemically bound to the compound by means of one or more covalent bonds. As used herein, "Covalent" includes the semicovalent bonds formed by chelate structures. "Chemical compound" as used herein, when referring to the compound of the invention, includes the entire compound including the radioactive isotope and all attached radicals and moieties. In general, the compound has no overall net positive charge at pH 12 and no net overall negative charge at pH 6.0. It is recognized that charge or ionization cannot always be entirely eliminated and when it is stated that the compound has no charge it is intended that less than one tenth of one numerical percent (0.1%) of the compound is in a charged form.

It is readily understood that as the pH of the medium increases, the compounds of the present invention, having a weakly basic moiety are gradually deprotonated and therefore assume a nonionized lipid soluble form which is usually due to the presence of lipophilic groups such as alkyl, cycloalkyl, arylalkyl, or arylcycloalkyl groups. Lowering the pH of the medium, on the other hand, results in increasing protonation of the weakly basic group resulting in increasing hydrophilicity of the compounds. These changes are reflected by the measured

partition coefficients and by behavior of the compounds in the biological systems which are described below as an integral part of the description of the present invention.

The ligand compounds of the invention, in addition to being intermediates to the formation of the radiopharmaceutical compounds of the invention, have a general utility as trace metal chelating agents. Thus, it is contemplated within the invention that the ligands are useful in chelating metals from metal processing solutions by the simple addition to such soluiton.

The following representative examples are provided to show preparation of the compounds of the invention and their radiopharmaceutical utilities.

Example I

Preparation of N-[2,3-Bis(t-butoxycarbamoyl)propanoyloxy]-succinimide

To a stirring suspension of 45 grams of ethyl 2,3-diamino-propanoate dihydrochloride in 300 ml of chloroform was sequentially added a solution of 36.88 grams of $NaHCO_3$ in 210 ml of $H_2O$, 87.7 grams of NaCl and 95.7 grams of di-t-butyl dicarbonate. The reaction mixture was refluxed for two hours, cooled to room temperature and the organic phase was recovered. The aqueous phase was extracted twice with chloroform (2 X 100 ml) and the organics were combined, dried over anhydrous $MgSO_4$ and concentrated to a solid. The material was recrystallized from carbon tetrachloride to provide 69.0 grams (95% yield) of the ethyl 2,3-bis(t-butoxycarbamoyl)propionate having a melting

point of 128°C.

To a suspension of 69.0 grams of ethyl 2,3-bis(t-butoxycarbamoyl) propionate in 150 ml ethanol and 250 ml $H_2O$, was added 17.3 grams of KOH. Upon completion of the reaction (monitored by TLC on silica gel, 30% ethyl acetate/methylene chloride), the reaction product was quenched with aqueous 2M HCl (130 ml) and the resulting solution was extracted with methylene chloride (3 X 200 ml). The organic extract was dried over anhydrous $Na_2SO_4$ and concentrated in vacuo to a syrup, which was treated with diethyl ether to form 52.5 grams of crystallized 2,3-Bis(t-butoxycarbamoyl) propionic acid.

To a cooled mixture containing 30 grams of 2,3-bis(t-butoxycarbamoyl)propionic acid and 12.48 grams of N-hydroxysuccinimide in 200 ml of methylene chloride was added a slurry of N, N'-dicyclohexylcarbodiimide (26.49 grams) in 100 ml of methylenechloride. The mixture was stirred, with cooling, for 10 minutes and thereafter at room temperature overnight. After 26 hours, the mixture was cooled and filtered and the precipitate discarded. The filtrate was washed with 100 ml of $H_2O$ and 2 X 80 ml of a saturated, aqueous $NaHCO_3$ solution. The organic extract was dried over anhydrous $MgSO_4$ and concentrated in vacuo to a syrup which was treated with hot diethyl ether, cooled, and the resulting crystallized material was filtered and dried. N-[2,3-bis(t-butoxycarbamoyl) propanoyloxy]-succinimide (34.52 grams) was recovered.

Example II

Preparation of 6-[N,N-diethylaminocarbonyl]-3,3,10,10-tetramethyl-1,2-dithia-5,8-diazacyclodeca-4,8-diene

Diethyl amine (30 ml) was added to a suspension of 21.78g (54.25 mmol) of the product of Example I in 100 ml of dry acetonitrile and the mixture was stirred with exclusion of moisture for 5 hours. The stirred mixture was cooled, filtered and the precipitate was washed with a small amount of cold acetonitrile and discarded. The filtrate was concentrated to a syrup which was dissolved in 200 ml of methylene chloride and thereafter washed with saturated sodium bicarbonate (2X75 ml). The organic extract was dried over anhydrous $Na_2SO_4$ and concentrated to give 2,3-bis(t-butoxycarbamoyl)-N,N-diethylpropanamide, as a thick syrup.

To a solution of diethyl ether (40 ml) saturated with dry HCl gas (at 25°C) was added a solution of the blocked amide [2,3-bis-(t-butoxycarbamoyl)N,N-diethylpropanamide] in diethyl ether (30 ml). HCl gas was bubbled through the resulting solution for 12-15 minutes during which time extensive precipitation of the deblocked compound, N,N-diethyl(2,3-diamino) propanamide dihydrochloride occurred. After the introduction of HCl gas was terminated, the mixture was stirred for 15 more minutes. The precipitate was subsequently collected by filtration, washed with diethyl ether and added to a mixture of triethylamine (50 ml), anhydrous $Na_2SO_4$ (15 g) and 11.11 g (53.85 mmol) of bis(2-mercapto-2-methylpropanal) in 70 ml of toluene. The mixture was subsequently refluxed, using a Dean-Stark trap, for 3 hours. The

reaction mixture was allowed to cool to room temperature, filtered, and the filtrate was concentrated *in vacuo*, treated with diethyl ether and cooled. The solid obtained was collected by filtration, washed with cold diethyl ether and dried to give 7.84 g of the diimine having a mp of 127-129°C.

Calcd. for $C_{15}H_{27}N_3OS_2$: C, 54.68; H, 8.26; N, 12.75; S, 19.46.

Found: C, 54.71; H, 8.25; N, 12.64; S, 19.61.

## Example III

### Preparation of 6-[N,N-diethylamino]methyl-3,3,10,10-tetramethyl-1,2-dithia-5,8-diazacyclodecane

Sodium borohydride (1.37 g; 36.4 mmol) was added portionwise to a cold stirring solution of the diimine product of Example II (3.0 g; 9.1 mmol) in 60 ml of absolute ethanol. Stirring was continued at 0°C for 1 hour and then at room temperature overnight. After 15 hours, the reaction mixture was treated with water (100 ml) and saturated with sodium chloride. The resulting solution was extracted with methylene chloride (3X70 ml), the organic extracts were combined, dried over anhydrous $Na_2SO_4$ and concentrated to a residue. A solution of this residue in dry tetrahydrafuran (THF) (30 ml) was added dropwise to a solution of 1M $BH_3$·THF (100 ml) under nitrogen. The reaction mixture was refluxed (under $N_2$) overnight. After 20 hours, the reaction mixture was cooled in an ice bath, quenched with methanol (80 ml) and stirred overnight. Dry HCl was then bubbled through this solution (to pH 2) and the solution was concentrated *in vacuo* to a syrup. The syrup was then treated with a small amount of

ethanol and chilled to give a white solid. The white solid was collected by filtration, washed with cold ethanol and dried to yield 2.32 g of the hyrochloride salt of the titled product.

Example IV

Preparation of N-Methyl-N-(3-N',N'-dimethylamino)propyl-[3,3,10,10-tetramethyl-1,2-dithia-5,8-diazacyclodeca-4,8-diene]-6-carboxamide

To 100 ml of dry acetonitrile was added 3.71 g (31.92 mmol) of N,N,N'-trimethylpropane-1,3-diamine. The mixture was stirred for 2 hours, cooled to 0°C, and filtered. The filtrate was concentrated to a syrup which was dissolved in 200 ml of $CH_2Cl_2$ and subsequently washed with a saturated solution of sodium bicarbonate (3X60 ml). The organic extract was dried over anhydrous $MgSO_4$ and concentrated to give the blocked product, 2,3-bis(t-butoxycarbamoyl)-N-methyl-N-[(3-N',N'-dimethylamino)propyl]-propanamide, as a syrup. The syrup was then dissolved in 60 ml of ethyl acetate and dry HCl gas was bubbled through the resulting solution for 15 minutes. The resulting mixture was then stirred for 45 minutes, cooled and filtered to give the hydrochloride of the deblocked amide. The hydrochloride was washed with ethyl acetate and added to a mixture of triethylamine (40 ml), anhydrous $Na_2SO_4$ (5g) and 6.3g (30.5 mmol) of bis(2-mercapto-2-methylpropanal) in 100 ml of benzene. The mixture was refluxed for 2 1/2 hours, allowed to cool to room temperature and subsequently filtered. The filtrate was washed with a saturated solution of sodium bicarbonate (80

ml), dried over anhydrous $MgSO_4$ and concentrated to a minimum volume. The product crystallized on standing and was recrystallized from diethyl ether/methylene chloride to give, after drying, 3.43g (30%) of the titled product having a mp of 111-113°C.

Calcd. for $C_{17}H_{32}N_4OS_2$: C, 54.80; H, 8.66; N, 15.04; S, 17.21.

Found: C, 54.56; H, 8.43; N, 14.85; S, 17.45.

Example V

Preparation of N,N,N'-trimethyl-N'-[6-(3,3,10,10-tetramethyl-1,2-dithia-5,8-diazacyclodecyl)methyl]-1,3-propanediamine

Sodium borohydride (1.23 g, 32.5 mmol) was added dropwise to a stirring solution of 3.0 g (8,1 mmol) of the diamine of Example IV in 40 ml of absolute ethanol and stirring was continued overnight. After 18 hours, the reaction mixture was diluted with water (60 ml) and extracted with methylene chloride (3 X 60 ml). The combined organic extracts were dried over anhydrous sodium sulphate and concentrated to a syrup. The latter was chromatographed on 130 g of silica gel (with a gradient of 0-30% methanol-methylene chloride) to give 2.4 g of a chromatographically homogeneous syrup. The syrup was dissolved in 20 ml of dry THF and this solution was added dropwise to a solution of 1M $BH_3 \cdot THF$ (32 ml) in 45 ml of dry THF (under nitrogen). After completion of the addition, the reaction mixture was refluxed overnight. After 15 hrs, the reaction was cooled and quenched with aqueous 2M HCl (to pH 2). The pH of the mixture then adjusted to 10 (with NaOH). The mixture was

subsequently extracted with methylene chloride (2 X 75 ml) and the organic extract was dried over anhydrous sodium sulphate. This solution was then concentrated to a syrup which was then dissolved in anhydrous diethyl ether and converted to the hydrochloride salt by bubbling dry HCl through this solution. The white solid was collected by filtration and dried to give 2.67 g of the hydrochloride salt of the titled product.

Example VI

Preparation of 3,3,10,10-tetramethyl-1,2-dithia-5,8-diazacyclodeca-4,8-diene-6-(N,N-diisopropyl)-carboxamide

To a reaction mixture of 20.6 g (0.078 mol) of 2,3-diamino-(N,N-diisopropyl)-propionamide dihydrochloride produced by first reacting diisopropyl amine with the N-hydroxy succinimide activated ester of Example V and subsequently deprotecting the product with HCl gas, and 250 ml of triethylamine in 300 ml of benzene was added 17 g (0.08 mol) of $\alpha$, $\alpha'$-dithiodiisobutyraldehyde. The reaction mixture was refluxed with a Dean-Stark trap for 1 hour and then cooled to room temperature. The solid was filtered and the filtrate was evaporated to dryness. The reaction residue was subjected to silica gel chromatographic separation (2.5% $CH_3OH$ in $CH_2Cl_2$) to give 13.3 g of pure product. Recrystallization in hexane gave the titled product having a m.p. of 110-111°C.

Calcd. for $C_{17}H_{31}N_3OS_2$: C, 57.14; H, 8.68; N, 11.77.

Found: C, 57.09; H, 8.50; N, 11.69.

Example VII

Reduction of 3,3,10,10-tetramethyl-1,2-dithia-5,8-diazacyclodeca-4,8-diene-6-(N,N-diisopropyl)-carboxamide

To a solution of 3.57 g (0.01 mol) of 3,3,10,10-tetramethyl-1,2-dithia-5,8-diazacyclodeca-4,8-diene-6-(N,N-diisopropyl)-carboxamide in 100 ml of THF was added dropwise, 50 ml (0.05 mol) of 1M $BH_3 \cdot THF$ complex.

The reaction mixture was refluxed under nitrogen for 16 hours and then cooled to room temperature and then quenched by adding 20 ml of concentrated HCl. The solution was evaporated to dryness. The white residue was dissolved in 100 ml of $C_2H_5OH$ and 20 ml of concentrated HCl. The solution was refluxed for 2 hours cooled to room temperature, evaporated to dryness, and the solid residue was treated with 200 ml of water. The pH was adjusted to about 10 by adding NaOH pellets and the solution was extracted with 300 ml of $CH_2Cl_2$. The organics were dried over anhydrous $Na_2SO_4$, filtered and evaporated to dryness. The resulting clear residue was subjected to silica gel chromatographic separation (5% $CH_3OH$ in $CH_2Cl_2$) and gave 1.6 g (46%) of product.

Calcd. for $C_{17}H_{37}N_3S_2$: C, 58.79; H, 10.66; N, 12.11.

Found: C, 58.84; H, 10.43; N, 11.92.

Example VIII

Preparation of 3,3,10,10-tetramethyl-1,2-dithia-5,8-diazacyclodeca-4,8-diene-6-(N-phenyl piperazinyl)-carboxamide:

To a reaction mixture of 18 g (0.05 mol) of 2,3-diamino-(N-phenyl piperazinyl)-propionamide trihydrochloride produced as described in Example VI and 50 ml of triethylamine in 100 ml of benzene was added 10.3 g (0.05 mol) of $\alpha$, $\alpha'$-dithiodiisobutyraldehyde. The reaction mixture was refluxed for 3 hours and the resulting water was condensed in a Dean-Stark trap. After cooling to room temperature, the solid was filtered and washed with 50 ml of benzene. The combined benzene solution was evaporated to dryness. The residue was dissolved in 200 ml of dichloromethane, dried over $Na_2SO_4$, filtered and then evaporated to dryness to give a solid material which was recrystallized in ether-dichloromethane to provide 11.7 g (56%) of the above identified product having a m.p. of 176-178$^oC$.

Calcd. for $C_{21}H_{30}N_4OS_2$: C, 60.29; H, 7.17; N, 13.40.

Found: C, 60.22; H, 7.36; N, 13.31.

Example IX

Reduction of 3,3,10,10-tetramethyl-1,2-dithia-5,8-diazacyclodeca-4,8-diene-6-(N-phenyl piperazinyl)-carboxamide

To a solution of 8.16 g (0.02 mol) of 3,3,10,10-tetramethyl-1,2-dithia-5,8-diaza-cyclodeca-4,8-diene-6-(N-phenyl piperazinyl)-carboxamide in 200 ml of THF was added 150 ml (0.15 mol) of a 1 M solution of $BH_3 \cdot THF$ complex. The reaction mixture was refluxed under nitrogen for 16 hours. After cooling to room temperature, the reaction was quenched by adding 30 ml of ethanol and then was

bubbled with hydrochloride gas. Evaporation of the solvents gave a white solid, which was dissolved in 300 ml of water and treated with NaOH pellets to adjust the pH to 10. The solution was extracted two times with 250 ml of dichloromethane and the combined organic solution was dried over $Na_2SO_4$ and evaporated to dryness. The residual solid was subjected to chromatographic separation on silica gel (EtOAC as eluent) to give 5.6 g (68%) of product, having a m.p. of 102-103°C.

Calcd. for $C_{21}H_{36}N_4S_2$: C, 61.76; H, 8.82; N, 13.,73.

Found: C, 61.57; H, 8.73; N, 13.54.

Example X

Preparation of 3,3,10,10-tetramethyl-1,2-dithia-5,8-diazacyclodeca-4,8-diene-6-(N-benzyl piperazinyl)-carboxamide

To a mixture of 22.1 g (0.06 mol) of 2,3-diamino-(N-benzyl piperazinyl)-propionamide trihydrochloride produced as described in Example VI and 100 ml of triethyl amine in 150 ml of benzene was added 12.4 g (0.06 mol) of α,α'-dithiodiisobutyraldehyde. The reaction mixture was refluxed for 3 hours and the resulting water was condensed in a Dean-Stark trap. After cooling to room temperature, the solid was filtered and washed with benzene. The combined benzene solution was evaporated to dryness. The crude product was separated by chromatographic separation on silica gel (5% of $CH_3OH$ as eluent) to give 7.8 g (30%) of product, having a m.p. of 189-191°C.

Calcd. for $C_{22}H_{32}N_4OS_2$: C, 61.11; H, 7.41; N, 12.96.

Found: C, 60.96; H, 7.41; N, 12.64.

Example XI

Reduction of 3,3,10,10-tetramethyl-1,2-dithia-5,8-diazacyclodeca-4,8-diene-6-(N-benzyl piperazinyl)-carboxamide

To a solution of 6.5 g (0.015 mol) of 3,3,10,10-tetramethyl-1,2-dithia-5,8-diazacyclodeca-4,8-diene-6-(N-benzyl piperazinyl)-carboxamide in 150 ml of THF was added dropwise 175 ml (0.175 mol) of 1M solution of borane-THF complex. The reaction solution was refluxed for 5 hours and then cooled to room temperature and quenched by adding 15 ml of concentrated HCl. The solvent was removed in vacuo and the white solid residue was treated with 150 ml of water. The pH was adjusted to 10 by adding with NaOH pellets and extracted two times with 200 ml of $CH_2Cl_2$. The organics were dried over $Na_2SO_4$, filtered, and evaporated to dryness. A clear liquid material was obtained, which was separated by chromatographic separation on silica gel (5% $CH_3OH$ in $CH_2Cl_2$) to give 3.3 g (52%) of product.

Calcd. for $C_{22}H_{38}N_4S_2$: C, 62.56; H, 9.00; N, 13.27.

Found: C, 61.62; H, 8.95; N, 12.58.

Example XII

Radiolabeling of N,N'-bis(2-methyl-2-thiopropyl)-N",N"-diethyl-propane-1,2,3-triamine

A solution of sodium technetium-99m pertechnetate in saline (1mCi) was added to a vial containing 4 mg of N,N'-bis(2-methyl-2-thiopropyl)-N",N"-diethyl-propane-1,2,3-triamine in 10 ml of ethanol and the resulting solution was allowed to stand at room temperature for 10 minutes. Sodium borohydride (30 mg) was added

and the mixture was vortexed and subsequently heated at 80°C for 10 minutes. After cooling to room temperature, the reaction mixture was treated with 2 ml each of water and 50% ethyl acetate-hexane. The resulting mixture was vortexed and the organic layer was collected. After repeated extractions (3 times) of the aqueous layer, the combined organic extracts were dried over anhydrous $Na_2SO_4$ and filtered. The filtered solution was then condensed to dryness with a stream of nitrogen. The residue was redissolved in absolute ethanol.

Example XIII

Radiolabeling of 6-(N-phenylpiperazinyl)-3,3,10,10-tetramethyl-1,2-dithia-5,8-diazacyclodecane Using Buffered Stannous Chloride as the Reducing Agent

A stock solution of stannous chloride/sodium pyrophosphate was prepared by mixing 25 mg of sodium pyrophosphate and 0.25 ml of stannous chloride solution ($SnCl_2 \cdot 2H_2O$,10 m/ml of 0.1NHCl) in 25 ml of water. A mixture of 6-(N-phenyl piperazinyl)-3,3,10,10-tetramethyl-1,2-dithia-5,8-diazacylcodecane ligand (3 mg) and sodium technetium-99m pertechnetate (lmCi) and 0.5 ml of the stock solution was heated in a water bath at 80° for 15 minutes. To this was added a 50% mixture of saturated bicarbonate and ethyl acetate (2 ml). The resulting mixture was vortexed and the organic layer was collected. The vortexing procedure was repeated 3 times. The combined organic extracts were dried over anhydrous $Na_2SO_4$ and filtered. The filtered solution was condensed to dryness with a stream of nitrogen. The residue was redissolved in absolute ethanol.

Example XIX

## Preparation of Lyophilized Kit

A stock solution of stannous chloride/sodium pyrophosphate is prepared by the method of Example XIII. 0.5 ml of the stock solution is combined with 3 mg of N,N'-bis(2-methyl-2-thiopropyl)-N",N"-diethyl-propane-1,2,3-triamine in an open vial. The resulting solution is subjected to lyophilization at -20°C and the resulting vial of freeze dried product is capped under a nitrogen atmosphere.

Example XV

## Partition Coefficients

Various radiopharmaceutical compounds of the invention were prepared by the process of the invention and designated as Samples B-L, having the basic ligand structure of the invention with $R_1$ - $R_8$ varied as identified in Table I and complexed with technetium-99m. Sample A is a prior art 10,10,13,13-tetramethyl-11,12-dithia-8,15-diaza spiro [5.9] pentadecane-technetium-99m complex. Partition coefficients were measured by mixing the compound with 3 g each of 1-octanol and phosphate buffer (pH 7.0 or 7.4) in a test tube. The tube was vortexed 3 minutes at room temperature and then centrifuged for 5 min. Two weighted samples (0.59 each) from the 1-octanol and buffer layers were counted in a well counter. The partition coefficient was determined by calculating the ratio of cpm/g of octanol to that

of buffer. Samples from the octanol layer were repartitioned until consistent partition coefficient values were obtained.

Example XVI

Radiopharmaceutical Distribution

Sprague-Dawley male rats (220-300 g) were injected intravenously (femoral vein) with the compounds of samples A-L in 0.2 ml of 1:1 saline/ethanol solution (0.5-2 micro Ci) under light ether anaesthesia. At different time periods (2 min. and 15 min.) after the injection, the animals were sacrificed and the brain was excised, weighed and radiation counted in a dual-channel automatic gamma counter. The brain % dose was estimated by comparison of tissue counts to suitably diluted amounts of the injected material. The brain-to-blood concentration ratios were calculated for the % dose/gram of wet tissue and the results for samples A-L are tabulated in Table II.

Example XVII

Autoradiogram Comparison

Autoradiograms were made of Sprague-Dawley male rat brains of animals that were intravenously injected with 6-[N,N-diethylamino]methyl-3,3,10,10-tetramethyl-1,2-dithia-5,8-diazacylcodecane technetium-99m complex of the invention and 10,10,13,13-tetramethyl-11,12-dithia-8,15-diazaspiro-[5.9]pentadecane-technetium-99m complex of the prior art. The

autoradiograms were taken at 2 and 15 minute intervals after injection. As can be seen from the figures, the lack of clear definition of the second 15 minute autoradiogram taken using the prior art compounds is in vivid contrast to the sharp definition obtained and retained using the compounds of the invention.

TABLE I

| Sample | Technetium Complex Structure $R_3$ | $R_{1-2}$ | $R_{5-8}$ | $R_4$ | Partition Coefficient pH 7.0 | Partition Coefficient pH 7.4 |
|---|---|---|---|---|---|---|
| A | $-Spiro-C_5H_{10}(R_3,R_4)$ | H | $C_2H_5$ | - | 384 | 607 |
| B | $-CH_2N(C_2H_5)_2$ | H | $CH_3$ | H | 96 | 109 |
| C | $-CH_2N(CH_3)-(CH_2)_3N(CH_3)_2$ | H | $CH_3$ | H | 3.2 | 8.1 |
| D | $-CH_2-c-N(CH_2)_5$ | H | $CH_3$ | H | 283 | 346 |
| E | $-CH_2N(CH_3)-(CH_2)_3N(CH_3)_2$ | H | $C_2H_5$ | H | 154 | 292 |
| F | $-CH_2NHCH(CH_3)CH_2C_6H_5$ | H | $C_2H_5$ | H | 156 | 179 |
| G | $-CH_2NH-i-C_3H_7$ | H | $CH_3$ | H | 86 | 85 |
| H | $-6,4'-SPIRO-CH_2CH_2N(CH_3)CH_2CH_2$ $(R_3R_4)$ | H | $CH_3$ | - | 6.7 | 12.4 |
| I | $-(CH_2)_3N(C_2H_5)_2$ | H | $CH_3$ | $CH_3$ | 105 | 147 |
| J | $-CH_2N(CH_2C_6H_5)(CH_2)_3N(CH_3)_2$ | H | $CH_3$ | H | 128 | 188 |
| K | $-CH_2-c-[-N(CH_2)_4N-]C_6H_5$ | H | $CH_3$ | H | 19 | 22 |
| L | $-6,4'-SPIRO-CH_2CH_2N(CH_3)CH_2CH_2$ $(R_3R_4)$ | H | $C_2H_5$ | - | 518 | 1084 |

TABLE II

| SAMPLE | BRAIN % DOSE 2 MIN | 15 MIN | BRAIN/BLOOD RATIO 2 MIN | 15 MIN |
|---|---|---|---|---|
| A | 2.2 | 0.7 | 1.4 | 2.2 |
| B | 3.82 | 0.94 | 4.04 | 2.81 |
| C | 0.17 | 0.17 | 0.26 | 0.70 |
| D | 1.46 | 0.96 | 0.47 | 0.51 |
| E | 0.20 | 0.10 | 0.32 | 0.52 |
| F | 0.69 | 0.22 | 0.50 | 0.70 |
| G | 0.70 | 0.21 | 1.16 | 0.71 |
| H | 0.09 | 0.05 | 0.12 | 0.19 |
| I | 0.11 | 0.06 | 0.58 | 0.08 |
| J | 0.18 | 0.09 | 0.32 | 0.37 |
| K | 3.02 | 0.75 | 5.07 | 3.73 |
| L | 0.10 | 0.06 | 0.20 | 0.41 |

WE CLAIM:

- 1 -

A compound of the formula:

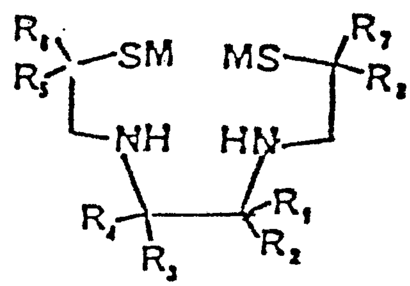

wherein each of $R_1$, $R_2$, $R_3$, $R_4$, $R_5$, $R_6$, $R_7$, and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; each M is selected from hydrogen and substituted or unsubstituted alkyl, alkene, acyl alkyl, acyl aryl, alkoxy alkyl, alkoxy aryl, acyl alkoxy, acyl aryloxy, silyl trialkyl and alkyl aryl, or M represents a covalent bond between the two sulfur atoms of the structure; provided at least one of $R_1$, $R_2$, $R_3$, $R_4$, $R_5$ $R_6$, $R_7$, and $R_8$ contains at least one nitrogen atom.

- 2 -

A compound of Claim 1 wherein at least one of $R_1$ - $R_8$ is selected from substituted or unsubstituted alkyl, aryl, cylcoalkyl, alkenyl and alkynyl and each M is selected from hydrogen, acyl, alkyl, acyl aryl and silyl trialkyl.

- 3 -

A compound of Claim 2 wherein at least one of $R_1$, $R_2$, $R_3$ and $R_4$ is selected from substituted or unsubstituted alkyl, aryl and cylcoalkyl.

- 4 -

A compound of Claim 3 wherein at least one of $R_1$, $R_2$, $R_3$ and $R_4$ is an alkyl containing an amine substitutent.

- 5 -

A compound of Claim 4 wherein said amine is selected from dialkyl amine and aryl amine.

- 6 -

A compound of Claim 5 wherein said aryl substituent contains at least one nitrogen, oxygen, phosphorus or sulfur substituted heterocyclic.

- 7 -

A compound of Claim 3 wherein at least one of $R_1$, $R_2$, $R_3$ and $R_4$ is aryl.

- 8 -

A compound of Claim 7 wherein said aryl contains a substituent selected from alkyl amine, and heterocyclic amine.

- 9 -

A compound of Claim 3 wherein at least one of $R_1$, $R_2$, $R_3$ and $R_4$ is selected from cycloalkyl.

- 10 -

A compound of Claim 9 wherein said cycloalkyl contains a substituent selected from alkyl amine and heterocyclic amine.

- 11 -

A compound of Claim 2 wherein at least one of $R_5$, $R_6$, $R_7$ and $R_8$ is selected from alkyl, aryl and cycloalkyl and contains a nitrogen substituent.

- 12 -

A compound of Claim 11 wherein said nitrogen substituent comprises a secondary, tertiary, or heterocyclic amine group.

- 13 -

A compound of Claim 1 wherein each of $R_5$, $R_6$, $R_7$ and $R_8$ is selected from methyl and ethyl.

- 14 -

A compound of Claim 13 wherein at least one of $R_1$, $R_2$, $R_3$ and $R_4$ is selected from the group consisting of N-[piperidino]methyl, N-[2-methyl piperidino]methyl, N-[4-methyl piperidino]methyl, N-[3,5-dimethyl piperidino]methyl, N-[3-methyl piperidino]methyl, N-[2,6-dimethylpiperidino]methyl, diethylaminomethyl, dimethylaminomethyl, isopropylaminomethyl, diisopropylaminomethyl, N-methylbenzylaminomethyl, $\alpha$-methylbenzylaminomethyl, N-methyl-N[3-N',N'-dimethylamino)-propyl]aminomethyl, -3-[2-(N-piperidino)ethoxy]phenyl,-3-[2-(N-morpholino)ethoxy]phenyl,-[2-(N-morpholino)ethoxy]methyl, N,N-diethylaminopropyl, N-[3-quinuclidyl]aminomethyl, -[N-methyl-N-allyl]aminomethyl, N-[phenylisopropyl]aminomethyl, aminomethyl and N-benzyl-N-[3-(N',N'-dimethylamino)propyl]methyl.

- 15 -

A compound of Claim 1 wherein each of $R_1$, $R_2$, $R_3$ and $R_4$ is selected from hydrogen, methyl and ethyl.

- 16 -

A compound of Claim 15 wherein at least one of $R_5$, $R_6$, $R_7$ and $R_8$ is selected from the group comprised of N-[piperidino]methyl, N-[2-methyl piperidino]methyl, N-[4-methyl piperidino]methyl, N-[3,5-dimethyl piperidino]methyl, N-[3-methyl piperidino]methyl, N-[2,6-dimethylpiperidino]methyl, diethylaminomethyl, dimethylaminomethyl, isopropylaminomethyl, diisopropylaminomethyl, N-methylbenzylaminomethyl, $\alpha$-methyl-

0200211

benzylaminomethyl, N-methyl-N[3-N',N'-dimethylamino)propyl]aminomethyl, -3-[2-(N-piperidino)ethoxy]phenyl,-3-[2-(N-morpholino)-ethoxy]phenyl,-[2-(N-morpholino)ethoxy]methyl N,N-diethylaminopropyl, N-[3-quinuclidyl]aminomethyl, -[N-methyl-N-allyl]-aminomethyl, N-[phenylisopropyl]aminomethyl, aminomethyl and N-benzyl-N-[3-(N',N'-dimethylamino)propyl]methyl.

- 17 -

A compound of the formula:

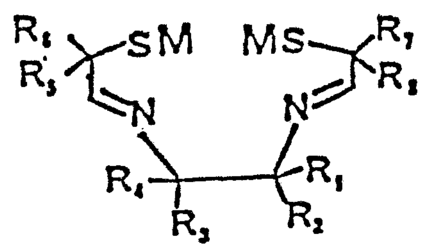

wherein each of $R_1$, $R_2$, $R_3$, $R_4$, $R_5$, $R_6$, $R_7$, and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; each M is selected from hydrogen and substituted or unsubstituted acyl alkyl, acyl aryl, silyl trialkyl and alkyl aryl, or M represents a covalent bond between the two sulfur atoms of the structure; provided at least one of $R_1$, $R_2$, $R_3$, $R_4$, $R_5$ $R_6$, $R_7$, and $R_8$ contains at least one nitrogen atom.

- 18 -

A compound of Claim 17 wherein at least one of $R_1$ - $R_8$ is selected from substituted or unsubstituted alkyl, aryl, cylcoalkyl, alkenyl and alkynyl.

- 19 -

A compound of Claim 17 wherein each M is selected from hydrogen, acyl alkyl, acyl aryl and silyl trialkyl.

- 20 -

A compound of Claim 18 wherein at least one of $R_5$, $R_6$, $R_7$ and $R_8$ is selected from substituted or unsubstituted alkyl and cycloalkyl.

- 21 -

A compound of Claim 17 wherein at least one of $R_1$, $R_2$, $R_3$ and $R_4$ is selected from substituted and unsubstituted alkyl, cycloalkyl, aryl, alkenyl and alkynyl.

- 22 -

A compound of the formula:

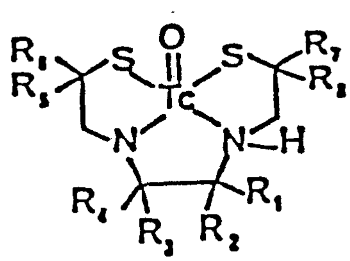

wherein each $R_1$, $R_2$, $R_3$, $R_4$, $R_5$, $R_6$, $R_7$, and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; provided at least one of $R_1$, $R_2$, $R_3$, $R_4$, $R_5$, $R_6$, $R_7$, and $R_8$, contains at least one nitrogen atom.

- 23 -

A compound of Claim 22 wherein at least one of $R_5$, $R_6$, $R_7$ and $R_8$ is selected from substituted or unsubstituted alkyl, aryl, cylcoalkyl, alkenyl and alkynyl.

- 24 -

A compound of Claim 23 wherein at least one of $R_5$, $R_6$, $R_7$ and $R_8$ contains a nitrogen atom.

- 25 -

A compound of Claim 22 wherein at least one of $R_1$, $R_2$, $R_3$ and $R_4$ is selected from substituted and unsubstituted alkyl, cycloalkyl, aryl, alkenyl and alkynyl.

- 26 -

A compound of Claim 25 wherein at least one of $R_1$, $R_2$, $R_3$ and $R_4$ contain a nitrogen atom.

A method for selectively depositing a radiopharmaceutical compound emitting radiation reading observable by radiation detecting means in at least one target tissue or organ of a mammal, the method comprising depositing a compound of the formula:

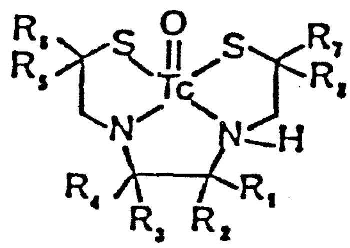

wherein each $R_1$, $R_2$, $R_3$, $R_4$, $R_5$, $R_6$, $R_7$, and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; provided at least one of $R_1$, $R_2$, $R_3$, $R_4$, $R_5$, $R_6$, $R_7$, and $R_8$, contains at least one nitrogen atom; into the bloodstream of the mammal, said compound having such acid base characteristics that a state of ionization of the compound at the pH of the blood of the mammal is significantly different than its state of ionization at the intracellular pH of the target tissue or organ, the compound having such lipid solubility characteristics that the compound is capable of ready penetration through cell walls of the target tissue or organ wherein due to a change in its state of ionization, its lipid solubility is substantially decreased whereby an ability of the compound to exit from the

target tissue or organ is substantially diminished so that the compound is at least temporarily trapped within the target tissue or organ; said compound having at least a 20% positive change in its n-octanol-aqueous medium partition coefficient at a pH change of from 6.60 to 7.60 pH units.

- 28 -

The method of Claim 27 wherein the compound contains at least one amine group.

- 29 -

The invention of Claim 27 wherein the partition coefficient of the compound has a positive charge of at least 50% in the range of 6.60 to 7.60 pH units.

- 30 -

A compound of Claim 29 wherein at least one of $R_5$, $R_6$, $R_7$ and $R_8$ is selected from substituted or unsubstituted alkyl, aryl, cylcoalkyl, alkenyl and alkynyl.

- 31 -

A compound of Claim 30 wherein at least one of $R_5$, $R_6$, $R_7$ and $R_8$ contains a nitrogen atom.

- 32 -

A compound of Claim 29 wherein at least one of $R_1$, $R_2$, $R_3$ and $R_4$ is selected form substituted and unsubstituted alkyl, cycloalkyl, aryl, alkenyl and alkynyl.

- 33 -

A compound of Claim 32 wherein at least one of $R_1$, $R_2$, $R_3$ and $R_4$ contain a nitrogen atom.

- 34 -

The pharmaceutical kit for manufacturing radiopharmaceutical compounds comprising a compound of the formual:

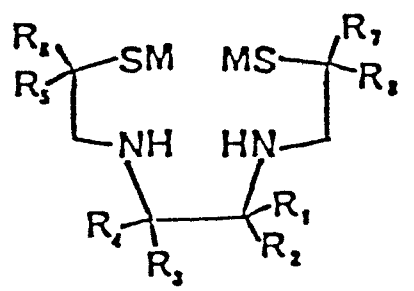

wherein each of $R_1$, $R_2$, $R_3$, $R_4$, $R_5$, $R_6$, $R_7$, and $R_8$, is independently selected from at least one of hydrogen and substituted or unsubstituted alkyl, alkenyl, alkynyl, cycloalkyl, heterocyclic alkyl, aryl, carboxylic acid ester, and carboxylic acid amide; each M is selected from hydrogen and substituted or unsubstituted acyl alkyl, acyl aryl, silyl trialkyl and alkyl aryl, or M represents a covalent bond between the two sulfur atoms of the structure; provided at least one of $R_1$, $R_2$, $R_3$, $R_4$, $R_5$ $R_6$, $R_7$, and $R_8$ contains at least one nitrogen atom; and a reducing agent.

- 35 -

A kit of Claim 34 additionally containing a buffer.

- 36 -

A kit of Claim 34 wherein the compound is lyophilized.

- 37 -

A kit of Claim 34 wherein the reducing agent is stannous chloride.

- 38 -

A kit of Claim 34 wherein each of $R_5$, $R_6$, $R_7$ and $R_8$ is selected from methyl and ethyl.

- 39 -

A kit of Claim 38 wherein at least one of $R_1$, $R_2$, $R_3$ and $R_4$ is selected from the group consisting of N-[piperidino]methyl, N-[2-methyl piperidino]methyl, N-[4-methyl piperidino]methyl, N-[3,5-dimethyl piperidino]methyl, N-[3-methyl piperidino]methyl, N-[2,6-dimethylpiperidino]methyl, diethylaminomethyl, dimethylaminomethyl, isopropylaminomethyl, diisopropylaminomethyl, N-methylbenzylaminomethyl, α-methylbenzylaminomethyl, N-methyl-N[3-N',N'-dimethylamino)propyl]-aminomethyl, -3-[2-(N-piperidino)ethoxy]phenyl,-3-[2-(N-morpholino)ethoxy]phenyl,-[2-(N-morpholino)ethoxy]methyl, N,N-diethylaminopropyl, N-[3-quinuclidyl]aminomethyl, -[N-methyl-N-allyl]aminomethyl, N-[phenylisopropyl]aminomethyl, aminomethyl and N-benzyl-N-[3-(N',N'-dimethylamino)propyl]methyl.

- 40 -

A kit of Claim 34 wherein each of $R_1$, $R_2$, $R_3$ and $R_4$ is selected from hydrogen, methyl and ethyl.

- 41 -

A kit of Claim 40 wherein at least one of $R_5$, $R_6$, $R_7$ and $R_8$ is selected from the group consisting of N-[piperidino]methyl, N-[2-methyl piperidino]methyl, N-[4-methyl piperidino]methyl, N-[3,5-dimethyl piperidino]methyl, N-[3-methyl piperidino]methyl, N-[2,6-dimethylpiperidino]methyl, diethylaminomethyl, dimethylaminomethyl, isopropylaminomethyl, diisopropylaminomethyl, N-methylbenzylaminomethyl, α-methylbenzylaminomethyl, N-methyl-N[3-N',N'-dimethylamino)propyl]aminomethyl, -3-[2-(N-piperidino)ethoxy]phenyl,-3-[2-(N-morpholino)ethoxy]phenyl,-[2-(N-morpholino)ethoxy]methyl, N,N-diethylaminopropyl, N-[3-quinuclidyl]aminomethyl, -[N-methyl-N-allyl]aminomethyl, N-[phenylisopropyl]aminomethyl, aminomethyl and N-benzyl-N-[3-(N',N'-dimethylamino)propyl]methyl.

- 42 -

A pharmaceutical composition comprising a pharmacologically suitable carrier and at least one compound of claim 1.

- 43 -

A pharmaceutical composition comprising a pharmacologically suitable carrier and at least one compound of claim 22.

- 44 -

A method of manufacturing a compound of Claim 1, wherein M represents a covalent bond between the two sulfur atoms of the structure, comprising reacting a compound of the formula:

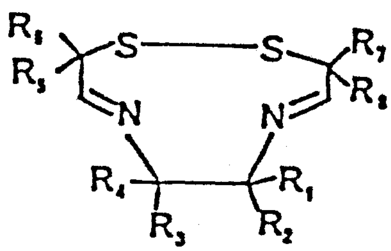

wherein $R_1$ - $R_8$ is as before described, with a strong reducing agent comprising at least one of aluminum or borane, in the presence of an appropriate non-aqueous solvent, at a temperature from above about room temperature to below about the reflux temperature of the solvent.

- 45 -

The method of Claim 44 wherein said compound of the formula:

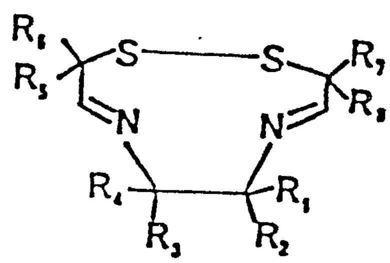

is prepared by reacting at a temperature of from about $50^0c$ to about $250^0c$, a dithio dialdehyde of the formula:

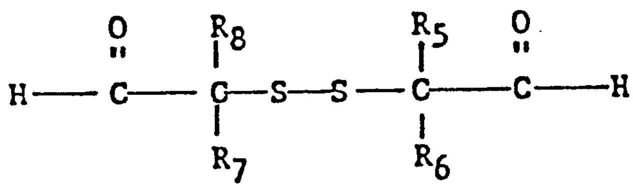

wherein $R_5$ - $R_8$ are as previously described, with a vicinal diamine of the formula:

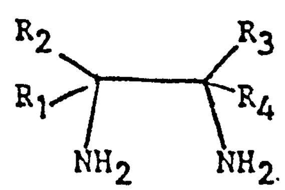

wherein $R_1$ - $R_4$ are as previously described, in the presence of an appropriate solvent which will remove water azeotropically.

- 46 -

The method of Claim 44 or 45 wherein said compound of the formula:

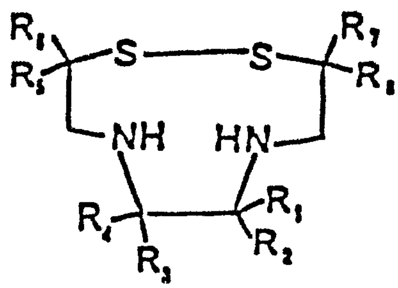

is thereafter radiolabeled by treatment with technetium -99m pertechnetate in the presence of a reducing agent.

- 47 -

The process of Claim 44 wherein said reducing agent comprises at least one of sodium borohydride or lithium aluminum hydride.

- 48 -

The process of Claim 44 wherein said solvent comprises at least one of benzene or toluene.

- 49 -

The process of Claim 45 wherein said dithio aldehyde and said vicinal diamine is reacted in the present of a solvent comprising at least one of benzene or toluene.

- 50 -

The method of Claim 46 wherein said reducing agent present with said pertechnetate comprises at least one of sodium borohydride, lithium aluminum hydride or stannous pyrophosphate.

- 51 -

The method of Claim 50 wherein said reduction is in the presence of ethanol or saline.

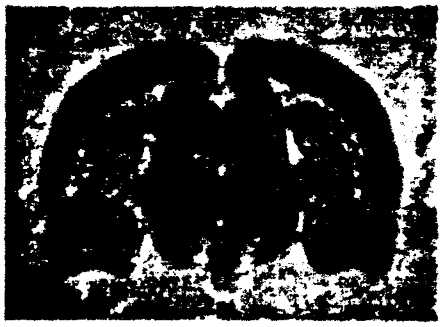

Fig. 1.

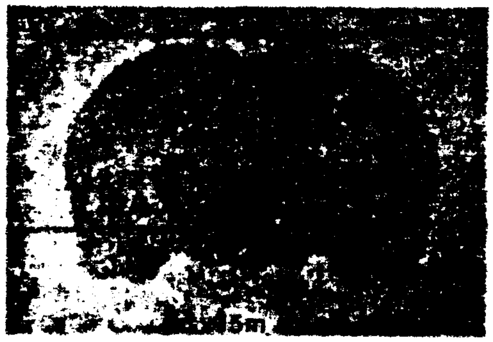



Fig. 2.

PRIOR ART

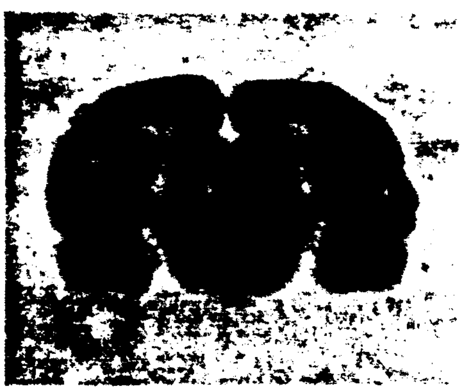

Fig. 3.

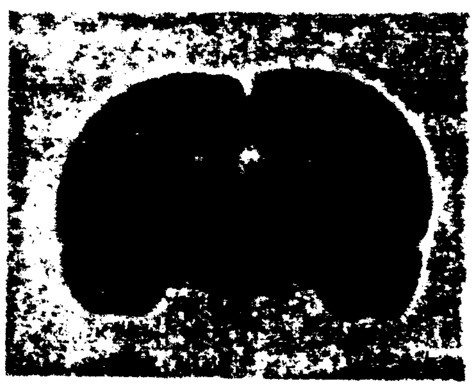

Fig. 4.

European Patent Office

**0200211**
Application number

# EUROPEAN SEARCH REPORT

EP 86 10 5920

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| Y | EP-A-0 135 160 (MASSACHUSETTS INSTITUTE OF TECHNOLOGY)<br>* Claims *<br>--- | 1,22,<br>27 | C 07 C 149/24<br>C 07 D 285/00<br>C 07 F 13/00<br>A 61 K 49/02 |
| Y | US-A-4 434 151 (E.F. BYRNE)<br>* Claims *<br>--- | 1,22,<br>27 | |
| P,X | EP-A-0 163 119 (THE JOHNS HOPKINS UNIVERSITY)<br>* Claims; pages 1,2 *<br>--- | 1,22,<br>27 | |
| P,X | JOURNAL OF MEDICINAL CHEMISTRY, vol. 28, no. 9, September 1985, pages 1280-1284, American Chemical Society, US; H.F. KUNG et al.: "Synthesis of new bis(aminoethanethiol)(BAT) derivatives: Possible ligands for 99mTc brain imaging agents"<br>* Pages 1280-1284 *<br>----- | 1-44 | **TECHNICAL FIELDS SEARCHED (Int Cl 4)**<br>C 07 C 149/00<br>C 07 D 285/00<br>C 07 F 13/00<br>A 61 K 49/00 |

The present search report has been drawn up for all claims

| Place of search | Date of completion of the search | Examiner |
|---|---|---|
| THE HAGUE | 11-08-1986 | MOREAU J.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO Form 1503 03 82